# EUROPÄISCHE PATENTSCHRIFT

(11) **EP 0 764 438 B1**
(45) Veröffentlichungstag und Bekanntmachung des Hinweises auf die Patenterteilung: **05.11.2008**
(21) Anmeldenummer: 96114361.7
(22) Anmeldetag: 07.09.1996
(51) Int. Cl.: A61K 8/92, A61Q 5/04

(54) **Mittel zur dauerhaften Verformung von menschlichen Haaren enthaltend die unverseifbare Bestandteile eines pflanzischen Öls**
Composition for permanent waving of human hair containing the non-soapable components of an vegetable oil
Composition pour l'ondulation permanente des cheveux humains contenant les composés non-savoneux d'une oil végétale

(30) Priorität: 19.09.1995 DE 19534723
(43) Veröffentlichungstag der Anmeldung: 26.03.1997
(73) Patentinhaber: KPSS-Kao Professional Salon Services GmbH, 64297 Darmstadt (DE)
(72) Erfinder: Rose, Burkhard, 64297 Darmstadt (DE); Nöcker, Bernd, Dr., 64372 Ober-Ramstadt (DE)

(56) Entgegenhaltungen:
- WO-A-94/21764
- DE-A- 2 019 226
- DE-A- 3 740 926
- FR-A- 2 679 448
- K. SCHRADER: "Der vorteilhafte Einsatz von Phytosterinen in kosmetischen Produkten" PARFÜMERIE UND KOSMETIK, Bd. 63, Nr. 1, 1982, Seiten 8, 10-18, XP002092566
- R. WACHTER ET AL: "Phytosterole- planzliche Wirkstoffe in der Kosmetik" PARFÜMERIE UND KOSMETIK, Bd. 75, Nr. 11, 1994, Seiten 755-761, XP002092567
- PATENT ABSTRACTS OF JAPAN vol. 13, no. 158 (C-586), 17. April 1989 & JP 63 316714 A (LION CORP), 26. Dezember 1988

## Beschreibung

Die vorliegende Erfindung betrifft ein Mittel zum Verformen von menschlichen Haaren, d.h. ein Dauerwellmittel, das eine ausgezeichnete Wellwirkung besitzt, insbesondere eine gleichmäßige Dauerwelle mit elastischen Locken und außergewöhnlich guter Kämmbarkeit ergibt, jedoch auf die Haarstruktur auch bei mehrfacher Anwendung keinerlei schädigende Wirkung ausübt und einen ausdrucksvollen Haarglanz bewirkt.

Die Dauerwellung erfordert bekanntlich zwei Behandlungsschritte:
Die reduktive Spaltung der Cystin-Disulfidbrücken des Haares durch Einwirkung eines Reduktionsmittels und die anschließende Neutralisierung bzw. Fixierung durch Aufbringung eines Oxidationsmittels, wodurch die Cystin-Disulfidbrücken wiederhergestellt werden.

Das überwiegend eingesetzte Reduktionsmittel ist auch heute noch Thioglykolsäure, insbesondere als Ammoniumsalz, obwohl zahlreiche andere Thio-Verbindungen für diesen Zweck vorgeschlagen wurden, die sich jedoch in der Praxis nicht durchgesetzt haben.

Die Thioglykolat enthaltenden Zusammensetzungen werden üblicherweise bei einem pH-Wert zwischen 8 und 10, insbesondere 8,5 bis 9,5, eingesetzt, was bei wiederholter, zeitlich nahe zusammenliegender Anwendung zu Haarschädigungen führen kann.

Man hat bereits versucht, diese Nachteile durch die Schaffung sogenannter "saurer Dauerwellmittel" zu überwinden, deren Anwendungs-pH-Wert bei etwa 6,8 bis 7,8, d.h. um den Neutralpunkt herum, liegt. Der in diesen Zusammensetzungen meistbenutzte reduzierende Wirkstoff ist der Thioglykolsäuremonoglycerinester. Es hat sich jedoch gezeigt, daß diese Substanz bei manchen Benutzerinnen hautreizend, insbesondere sensibilisierend, wirkt, so daß auch diese Lösung nicht optimal ist.

Es wurde nunmehr gefunden, daß diese Nachteile überwunden werden können und ein Dauerwellmittel auf Basis mindestens einer reduzierenden Thioverbindung, das eine gleichmäßige Wellwirkung besitzt und die Haarstruktur in keiner Weise schädigt, sondern dem dauergewellten Haar Glanz und eine außergewöhnlich gute Kämmbarkeit verleiht, dadurch hergestellt werden kann, wenn man diesem Mittel 0,01 bis 2,5 Gew.-%, insbesondere 0,05 bis 1 Gew.-%, bezogen auf die Gesamtmenge des Mittels, der unverseifbaren Bestandteile mindestens eines pflanzlichen Öls zusetzt, die mindestens 20, vorzugsweise mindestens 25 Gew.-% Phytosterole, enthalten.

Besonders bevorzugt ist ein Bereich von etwa 20 bis etwa 40, insbesondere etwa 30 Gew.-% Phytosterol, bezogen auf die unverseifbaren Bestandteile des pflanzlichen Öls oder eines eingesetzten Gemisches pflanzlicher Öle.

Die erfindungsgemäß verwendeten unverseifbaren Bestandteile dieser Öle enthalten vorzugsweise etwa 30 bis etwa 60, insbesondere 40 bis etwa 55, z. B. 50 Gew.-% Triglyceride sowie weitere Bestandteile.

Geeignete Öle, deren unverseifbare Bestandteile eingesetzt werden, sind insbesondere Avocadoöl, dessen unverseifbarer Teil unter der Handelsbezeichnung Avocadin® bekannt ist, Sesamöl, Sonnenblumenöl, Mandelöl, Pfirsichkernöl, Weizenkeimöl, Macadamianußöl, Nachtkerzenöl, Jojobaöl, Ricinusöl oder auch Oliven- bzw. Sojaöl.

Die erfindungsgemäßen Dauerwellmittel enthalten mindestens eine reduzierende Thioverbindung. Bevorzugt sind Thioglykolsäure und Thiomilchsäure sowie deren Salze, insbesondere die Ammonium- und Ethanolaminsalze.

Weitere einsetzbare Thioverbindungen sind insbesondere Cystein bzw. dessen Hydrochlorid, Monocystein, Cysteamin, N-Acetylcystein, Thioglycerin, Ethandiolmonothioglykolat, 1,2-Propylenglykolmonothioglykolat (vgl. auch WO-A 93/1791), 1,3-Propandiolmonothioglykolat bzw. das daraus resultierende Isomerengemisch, 1,3-Butandiol- und 1,4-Butandiolmonothioglykolat bzw. deren Isomerengemische, Ethandiolmonothiolactat, 1,2-Propandiol- und 1,3-Propandiolmonothiolactat und deren Isomerengemische, 1,3-Butandiol- und 1,4-Butandiolmonothiolactat und deren Isomerengemische, Polyethylenglykol- wie Di-, Tri- und Tetraethylenglykolmonothioglykolate und -monothiolactate, Polypropylenglykol- wie Di-, Tri- und Tetrapropylenglykolmonothiolactate und -monothioglykolate, Glycerinmonothiolactate und weitere Thiosäuren und deren Ester sowie Gemische derselben.

Der Gesamtgehalt an Reduktionsmitteln in den erfindungsgemäßen Zusammensetzungen beträgt üblicherweise 2,5 bis etwa 15 Gew.-%, berechnet auf freie Thioglykolsäure als Bezugssubstanz.

Die Reduktionsmittel enthaltenden Dauerwellpräparate können, falls erforderlich, einen Gehalt an Alkalisierungsmitteln aufweisen. Die Menge ist abhängig vom reduzierenden Wirkstoff und dem angestrebten pH-Wert der Zusammensetzung. Vorzugsweise enthält die Reduktionsmittel-Zusammensetzung etwa 0,1 bis etwa 5, insbesondere etwa 0,5 bis etwa 2,5 Gew.-% desselben.

Bevorzugte Alkalisierungsmittel im Rahmen der Erfindung sind Ammoniumcarbamat, Ammoniak und/oder Ammonium(bi)carbonat. Es wird die Einstellung eines pH-Wertes im Bereich zwischen etwa 6,5 und etwa 9,5, vorzugsweise etwa 7 bis 8,5, angestrebt.

Die erfindungsgemäß zum Einsatz kommenden Dauerwellmittel enthalten vorzugsweise auch Tenside. Deren Anteil liegt bei etwa 0,1 bis etwa 10, insbesondere etwa 1 bis etwa 5 Gew.-%, der das Reduktionsmittel enthaltenden Zusammensetzung.

Sowohl bei den in den Reduktionsmittel-Zusammensetzungen als auch bei den in den Fixiermitteln eingesetzten Tensiden handelt es sich vorzugsweise um die bekannten anionaktiven Produkte, die gegebenenfalls auch in Kombination mit nichtionischen Tensiden zum Einsatz gelangen.

Geeignete anionische Tenside sind besonders die bekannten Alkylethersulfate und -carbonsäuren, insbesondere in Form ihrer Alkalisalze, sowie Eiweiß-Fettsäure-Kondensate.

Geeignete nichtionische Tenside sind insbesondere C₈-C₁₈-Fettalkoholpolyglykolether, Fettsäurepolyglykolester, Fettsäurealkanolamide, Aminoxide und vor allem C₈-C₁₈-Alkylpolyglukoside.

Es können auch amphotere Tenside wie die bekannten Betaine und Amidobetaine sowie, insbesondere in kationischen Fixierungen, kationaktive Tenside wie quaternäre Ammoniumverbindungen eingesetzt werden.

Ein weiterer wünschenswerter Bestandteil der erfindungsgemäß verwendeten Reduktionsmittel-Zusammensetzungen ist ein C₃-C₆-Alkandiol bzw. dessen Ether, insbesondere Mono-C₁-C₃-alkylether.

Bevorzugte Substanzen sind in diesem Zusammenhang 1,2- und 1,3-Propandiol, 1-Methoxypropanol(-2), 1-Ethoxypropanol(-2), 1,3- und 1,4-Butandiol, Diethylenglykol und dessen Monomethyl- und Monoethylether sowie Dipropylenglykol und dessen Monomethyl- und Monoethylether.

Der Anteil dieser Diole liegt vorzugsweise zwischen 0,5 und 30, vorzugsweise etwa 1 bis etwa 15, insbesondere etwa 5 bis etwa 10 Gew.-% der Reduktionsmittel-Zusammensetzung.

Neben den C₃-C₆-Alkandiolen bzw. deren Ethern können zusätzlich auch Monoalkohole wie Ethanol, Propanol-1, Propanol-2 sowie Polyalkohole wie Glycerin und Hexantriol, Ethylcarbitol, Benzylalkohol, Benzyloxyethanol sowie Propylencarbonat (4-Methyl-1,3-dioxolan-2-on), N-Alkylpyrrolidone und Harnstoff Verwendung finden.

Besonders bevorzugte zusätzliche Bestandteile sind anionische, kationische, nichtionische und amphotere Polymere, insbesondere kationische Polymere, vorzugsweise in einer Menge von etwa 0,25 bis etwa 5, insbesondere etwa 0,5 bis 2,5 Gew.-% der Gesamtzusammensetzung des Wellmittels.

Geeignete Polymere sind insbesondere solche des Typs "Polyquaternium" nach dem "CTFA International Cosmetic Ingredient Dictionary", 4th Ed.

Die erfindungsgemäß eingesetzten Mittel können selbstverständlich alle in Dauerwellmitteln üblichen Stoffe enthalten, auf deren detaillierte Aufzählung hier verzichtet wird, und als (wäßrige) Lösungen, Emulsionen, Cremes, Schäume etc. vorliegen.

Zur Vermeidung von Wiederholungen wird hierzu auf den Stand der Technik verwiesen, wie er beispielsweise in "Ullmann's Encyclopedia of Industrial Chemistry", Vol. A12 (1986), S. 588 bis 591, sowie insbesondere in der Monographie von K. Schrader, "Grundlagen und Rezepturen der Kosmetika", 2. Auflage (1989, Hüthig-Verlag), S. 823 bis 840, sowie in dem Übersichtsartikel von D. Hollenberg et al. in "Seifen-Öle-Fette-Wachse" 117 (1991), S. 81 - 87, beschrieben ist.

Die dort geoffenbarten Zusammensetzungen und Einzelbestandteile, auf die ausdrücklich Bezug genommen wird, können auch im Rahmen der vorliegenden Erfindung verwendet werden.

Falls erwünscht, kann vor dem Auftrag des Reduktionsmittels noch ein Vorbehandlungsmittel appliziert werden, wie es beispielsweise in der DE-A 37 40 926 beschrieben ist. Nach dem Aufbringen dieses Vorbehandlungsmittels wird das Haar aufgewickelt und die Reduktionsmittel-Zusammensetzung aufgetragen. Nach etwa 15- bis 30-minütiger Einwirkung und Spülung erfolgt die Fixierung mit Behandlung durch die üblichen und aus dem Stand der Technik hinreichend bekannten Peroxid- oder Bromat-Zusammensetzungen.

Ebenso kann selbstverständlich eine an sich bekannte Zwischenbehandlung zwischen Reduktions- und Neutralisationsphase erfolgen.

Die folgenden Beispiele dienen der Illustration der Erfindung.

### Beispiel 1

| Lösung | |
|---|---|
| Ammoniumthioglykolat (61%-ig) | 21,60 (Gew.-%) |
| Ammoniumhydrogencarbonat | 5,00 |
| Chlorophyllin | 0,02 |
| Proteinhydrolysat | 0,40 |
| Cocoamidopropylbetain | 0,50 |
| 1,2-Propandiol | 2,00 |
| Laureth-23 | 1,00 |
| Oleth-5 | 0,50 |
| Avocadin® | 0,20 |
| Parfumöl | 0,40 |
| Wasser | @ 100,00 |
| mit NH₃ eingestellt auf pH 8,5. | |

Die Dauerwellung erfolgte durch 20- bis 30-minütige Einwirkung auf das auf Lockenwickler gewickelte Haar, Ausspülen und anschließende Fixierung mit der folgenden Zusammensetzung:

| | |
|---|---|
| Wasserstoffperoxid | 2,5 (Gew.-%) |
| Cetylstearylalkohol | 2,0 |
| Natriumlaurylethersulfat | 1,2 |
| C₁₂-C₁₄-Alkylpolyglykolether | 1,0 |
| Stabilisator, Parfumöl | q.s. |
| Wasser | @ 100,0 |

Es wurde ein glänzendes, elastisch verformtes, leicht kämmbares Haar erhalten; eine Hautreizung wurde auch bei wiederholter Anwendung nicht beobachtet.
Weglassen des Avocadins führte zu einer weniger elastischen, etwas stumpfen Dauerwelle mit verschlechterter Kämmbarkeit, was insbesondere im Halbseitenversuch deutlich erkennbar war.

### Beispiel 2

| Gel | |
|---|---|
| Ammoniumthioglykolat (61%-ig) | 20,0 (Gew.-%) |
| Ammoniumhydrogencarbonat | 3,5 |
| Kationisches Polymer (Polyquaternium-2) | 0,5 |
| C₈-C₁₀-Alkylpolyglucosid (P.D.: ∼ 1,4) | 0,6 |
| Quaternium-18 | 0,1 |
| Myristylalkohol | 0,7 |
| Oleylalkohol | 0,2 |
| Paraffinöl | 0,8 |
| Bisabolol | 0,1 |
| Unverseifbare Bestandteile des Avocadoöls (∼ 30 % Phytosterol; ∼ 50 % Triglyceride) | 0,4 |
| Ceteareth-25 | 0,8 |
| PEG-40 hydriertes Ricinusöl | 0,6 |
| Laureth-23 | 0,6 |
| Parfumöl | 0,3 |
| Wasser | @ 100,0 |
| mit NH₃ eingestellt auf pH 8,2 | |

Es wurde eine ähnlich strukturierte Dauerwellung wie mit der Zusammensetzung nach Beispiel 1 erhalten.

### Beispiel 3

| Creme | |
|---|---|
| Ammoniumthioglykolat (61%-ig) | 18,0 (Gew.-%) |
| Anmoniumhydrogencarbonat | 2,5 |
| Kationisches Polymer (Polyquaternium-4) | 0,2 |
| Polyoxyethylen(5)stearylstearat | 4,0 |
| Steareth-21 | 2,0 |
| Paraffinöl | 3,0 |
| Polyoxyethylen(30)sorbitol | 3,0 |
| Mandelöl | 1,0 |
| Unverseifbare Bestandteile des Avocadoöls (∼ 30 % Phytosterol) | 1,5 |
| Tocopherolacetat | 0,5 |
| Parfumöl | 0,4 |
| Wasser | @ 100,0 |
| mit NH₃ eingestellt auf pH 8,8. | |

### Beispiel 4

| Zweiphasen-Produkt (Neutral-Dauerwelle) | |
|---|---|
| Teil A: | |
| Ammoniumhydrogencarbonat | 4,5 (g) |
| Ethanol | 0,5 |
| Diethylenglykolmonoethylether | 1,0 |
| Kationisches Polymer (Polyquaternium-22) | 1,0 |
| Laureth-23 | 1,0 |
| Oleth-5 | 0,5 |
| Unverseifbare Bestandteile des Avocadoöls (∼ 30 % Phytosterol; ∼ 50 % Triglyceride) | 0,2 |
| Wasser | @ 72,2 |
| eingestellt mit NH₃ auf pH 8,4 | |

| Teil B: | |
|---|---|
| Ammoniumthioglykolat, 70%-ig | 18,5 (g) |
| Thiomilchsäure | 2,0 |
| Cysteinhydrochlorid ₓ H₂O | 3,0 |
| 1,2-Propandiol | 0,5 |
| Wasser | @ 27,8 |
| eingestellt mit NH₃ auf pH 5,55. | |

Die Teilzusammensetzungen A und B wurden getrennt in ein bekanntes Zweikammerbehältnis eingebracht und unmittelbar vor der Anwendung auf das Haar durch Zerstören der Trennwand vereinigt.

Es wurde ein Produkt mit einem pH-Wert von 7,45 erhalten.

Nach üblicher Dauerwellbehandlung, Zwischenbehandlung und Fixierung wurde eine elastische Dauerwellung erreicht. Das behandelte Haar zeigte einen hellen Glanz und ließ sich ausgezeichnet kämmen.

Hautirritationen oder -sensibilisierungen traten nicht auf.

## Patentansprüche

1. Mittel zur dauerhaften Verformung von menschlichen Haaren, enthaltend in wäßriger Grundlage mindestens eine reduzierende Thioverbindung sowie 0,01 bis 2,5 Gew.-%, berechnet auf die Gesamtzusammensetzung des Mittels, der unverseifbaren Bestandteile mindestens eines pflanzlichen Öls mit einem Gehalt von mindestens 20 Gew.-% Phytosterol.

2. Mittel nach Anspruch 1, enthaltend 0,05 bis 1 Gew.-% der unverseifbaren Bestandteile mindestens eines pflanzlichen Öls mit einem Gehalt an mindestens 25 Gew.-% Phytosterol.

3. Mittel nach Anspruch 1 und/oder 2, **dadurch gekennzeichnet, daß** die unverseifbaren Bestandteile des pflanzlichen Öls mindestens 50 Gew.-% Triglyceride enthalten.

4. Mittel nach Anspruch 1, **dadurch gekennzeichnet, daß** es die unverseifbare Fraktion des Avocadoöls enthält.

5. Mittel nach einem oder mehreren der Ansprüche 1 bis 4, **dadurch gekennzeichnet, daß** es 0,25 bis 5 Gew.-%, berechnet auf die Gesamtzusammensetzung des Mittels, mindestens eines kationischen Polymeren enthält.

## Claims

1. Composition for permanent waving of human hair, comprising at least one reducing thio compound and from 0.01% to 2.5% by wt., calculated to the total composition, of the unsaponifiable ingredients of at least one vegetable oil having a phytosterol content of at least 20% by wt.

2. Composition according to claim 1, comprising from 0.05% to 1% by wt. of the unsaponifiable ingredients of at least one vegetable oil having a total phytosterol content of at least 25% by wt.

3. Composition according to claim 1 and (or) 2, comprising the unsaponifiable ingredients of the vegetable oil containing at least 50% by wt. triglycerides.

4. Composition according to claim 1, containing the unsaponifiable fraction of avocado oil.

5. Composition according to one or more of claims 1 to 4, containing from 0.25% to 5% by wt., calculated to the total composition, of at least one cationic polymer.

## Revendications

1. Produit pour l'ondulation permanente des cheveux humains, contenant dans une base aqueuse, au moins un composé thio réducteur ainsi que 0,01 à 2,5% en poids, calculés par rapport à la composition totale du produit, de constituants non saponifiables d'au moins une huile végétale ayant une teneur en phytostérol d'au moins 20% en poids.

2. Produit selon la revendication 1, contenant 0,05 à 1% en poids de constituants non saponifiables d'au moins une huile végétale ayant une teneur en phytostérol d'au moins 25% en poids.

3. Produit selon la revendication 1 et/ou 2, **caractérisé en ce que** les constituants non saponifiables de l'huile végétale contiennent au moins 50% en poids de triglycérides.

4. Produit selon la revendication 1, **caractérisé en ce qu'**il contient la fraction non saponifiable de l'huile d'avocat.

5. Produit selon l'une ou plusieurs des revendications 1 à 4, **caractérisé en ce qu'**il contient 0,25 à 5% en poids, calculés par rapport à la composition totale du produit, d'au moins un polymère cationique.
